# EUROPEAN PATENT APPLICATION

(11) **EP 4 822 312 A2**
(43) Date of publication of application: **14.10.2026**
(21) Application number: 26180566.7
(22) Date of filing: 14.03.2023
(51) Int. Cl.: G01J 3/10

(54) **SYSTEM OF EGG FERTILITY AND GENDER DETECTION**

(30) Priority: 14.03.2022 US 202263319539 P
(62) Document number(s) of the earlier application(s) in accordance with Art. 76 EPC: 23769391.6 / 4 493 915
(71) Applicant: Matrixspec Solutions Inc., Baie D'Urfé, QC H9X 3V1 (CA)
(72) Inventor: LIU, Li, Baie D'Urfé, H9X 3V1 (CA); NGADI, Michael, Baie D'Urfé, H9X 3V1 (CA)
(74) Representative: Barker Brettell LLP

(57) **Abstract**

An egg analysis system and methods are provided. The system comprises a light source for illuminating an egg, a hyperspectral imaging camera, a processor, and a memory storing instructions which, when executed by the processor, configure the processor to actuate the light source to illuminate the egg, receive images of the egg from the hyperspectral imaging camera, and analyze the images. The light source may emit light at one of approximately 860 nm, 1050 nm or 1250 nm.

## Description

### FIELD

The present disclosure generally relates to systems and methods of detecting egg fertility and gender.

### INTRODUCTION

Fertility and hatchability of eggs are critical economic factors for hatcheries and poultry breeding farms. Only about 60 to 90% of incubated eggs are fertile and eventually hatch in commercial hatcheries. Non-hatching eggs include infertile eggs or fertile eggs where the embryos have died. Infertile eggs may comprise up to 25% of all eggs set. These eggs may be useful for commercial egg tables or low grade food stock.

The sex of fertile eggs is also among the egg characteristics of interest for the poultry industry. In the layer egg industry, chicks are sexed at hatch and the female birds (that will lay eggs) are considered paramount while the male birds are culled. The opposite is the case with the broiler industry in which the male species are crucial. In either case, discarding of the unwanted chicks creates serious bottlenecks as far as waste disposal and animal welfare issues are concerned.

### SUMMARY

In accordance with an aspect, there is provided an egg analysis system. The system comprises system comprises a light source for illuminating an egg, a hyperspectral imaging camera, a processor, and a memory storing instructions which, when executed by the processor, configure the processor to actuate the light source to illuminate the egg, receive images of the egg from the hyperspectral imaging camera, and analyze the images.

In accordance with another aspect, the light source may emit light at one of approximately 860 nm, 1050 nm or 1250 nm.

In accordance with another aspect, there is provided a computer-implemented method of determining fertility and sex of eggs. The computer-implemented method comprises receiving angle, frequency (or wavelength) and intensity data of scattering light from an egg illuminated using a light source, identifying a germinal disc in the egg from the scattering light data, and determining at least one of a fertility or a sex of the egg based on the size and structure of the germinal disc

In accordance with another aspect, there is provided a computer-implemented method of determining fertility and sex of eggs. The computer-implemented method comprises receiving angle, frequency and intensity data of scattered light, fluorescence from an egg illuminated using a laser (i.e., a continuous wave, pulsed or supercontinuum laser), measuring the scattering light data to determine the particle size and density of a germinal disc of the egg, determining an average scattering size of the germinal disc, determining average cellular/particles size of germinal disc, a structure of the germinal disc, and determining a fertility and a sex of the egg based on the size and structure of the germinal disc.

In various further aspects, the disclosure provides corresponding systems and devices, and logic structures such as machine-executable coded instruction sets for implementing such systems, devices, and methods.

In this respect, before explaining at least one embodiment in detail, it is to be understood that the embodiments are not limited in application to the details of construction and to the arrangements of the components set forth in the following description or illustrated in the drawings. Also, it is to be understood that the phraseology and terminology employed herein are for the purpose of description and should not be regarded as limiting.

Aspects of the invention are described in the following numbered clauses:

1. An egg analysis system comprising:
a light source for illuminating an egg;
a hyperspectral imaging camera;
a processor; and
a memory storing instructions which, when executed by the processor, configure the processor to:
   actuate the light source to illuminate the egg;
   receive images of the egg from the hyperspectral imaging camera; and analyze the images.
2. The system as described in clause 1, wherein the light source provides a light that can cover the field of view (FOV) of the hyperspectral camera.
3. The system as described in clause 2, wherein the FOV is approximately 16 centimeters x 2.5 centimeters.
4. The system as described in clause 1, wherein the light source provides a high spectral intensity light source at the wavelengths of the light source.
5. The system as described in clause 4, wherein the wavelength range is at least 120 mW·sr⁻¹·cm⁻²·nm⁻¹ for spectral intensity.
6. The system as described in clause 1, wherein the light source provides a uniform illumination for each egg within the FOV.
7. The system as described in clause 6, wherein a variance of the uniform illumination is less than ten percent.
8. The system as described in clause 1, wherein the light source provides a 'cool' light that produces little heat for a long time run.
9. The system as described in clause 1, wherein the light source provides high stability for a long time run.
10. The system as described in clause 9, wherein the long time run is approximately 8 hours or above.
11. The system as described in clause 1, wherein the light source provides a long lifetime of the lamp.
12. The system as described in clause 11, wherein the long lifetime is greater than 1,000 hours.
13. The system as described in clause 1, wherein the light source comprises two or more halogen lamps wherein each halogen lamp comprises:
   a 250 W QTH lamp;
   a light source housing;
   a lamp power supply;
   a fiber bundle focusing assembly;
   a liquid light guide; and
   a light guide collimating probe.
14. The system as described in clause 1, wherein the light source comprises a light emitting node emitting light at a wavelength comprising one of approximately:
   860 nanometers;
   1050 nanometers; or
   1250 nanometers.
15. The system as described in clause 1, comprising:
   a photon detector for detecting a fluctuation of scattering light emitted from the egg; and
   wherein the at least one processor is configured to:
      receive scattering light data from the photon detector;
      digitize the scattering light data; and
      analyze the digitized scattering light data.
16. The system as described in clause 15, wherein the at least one processor is configured to:
   receive angle, frequency and intensity data of the scattering light;
   identify a germinal disc of the egg; and
   determine at least one of a fertility or a sex of the egg based on the size and structure of the germinal disc.
17. The system as described in clause 16, wherein to identify the germinal disc of the egg, the at least one processor is configured to:
   analyze light scattering data to identify features of material present; and
   compare the identified features of material present with known features for germinal discs.
18. The system as described in clause 16, wherein to determine the fertility of the egg, the at least one processor is configured to compare features of the identified germinal disc with known fertility features for germinal discs.
19. The system as described in clause 16, wherein to identify the germinal disc, the at least one processor is further configured to:
   receive the egg in a tray;
   actuate the tray to position the egg at a first angle for a first period of time, with a large end of the egg higher than a small end of the egg; and
   actuate the tray to position the egg in a vertical position with the large end up for a second period of time.
20. The system as described in clause 19, wherein at least one of:
   the first angle is approximately 45 degrees;
   the first period is approximately 24 hours; or
   the second period is approximately 48 hours.

Many further features and combinations thereof concerning embodiments described herein will appear to those skilled in the art following a reading of the instant disclosure.

### DESCRIPTION OF THE FIGURES

Embodiments will be described, by way of example only, with reference to the attached figures, wherein in the figures:
**FIG. 1A** illustrates, in a component diagram, an example of a system for analyzing eggs, in accordance with some embodiments;
**FIG. 1B** illustrates, in a component diagram, another example of a system for analyzing eggs in accordance with some embodiments;
**FIG. 2A** illustrates, in a schematic drawing, an example of a system with optical fiber with incident beam angle for acquiring scattering images of eggs, in accordance with some embodiments;
**FIG. 2B** illustrates, in a schematic drawing, an example of a system with LED line light for acquiring scattering images of eggs, in accordance with some embodiments;
**FIGs. 3A to 3E** illustrate scan images of a germinal disc in an egg, in accordance with some embodiments;
**FIG. 4** illustrates, in a flowchart, an example of a method of analyzing eggs, in accordance with some embodiments;
**FIG. 5** illustrates, in a flowchart, an example of a protocol (e.g., method) of handling an egg such that its germinal disc is located on the top (large end) of the egg, in accordance with some embodiments;
**FIG. 6A** illustrates an example of eggs positioned at 45 degree angle, in accordance with some embodiments;
**FIG. 6B** illustrates an example of eggs positioned in a vertical position, in accordance with some embodiments;
**FIG. 7** illustrates, in a flowchart, an example of a method of analyzing light scattering, in accordance with some embodiments; and
**FIG. 8** is a schematic diagram of a computing device such as a server or other computer in a device.

It is understood that throughout the description and figures, like features are identified by like reference numerals.

### DETAILED DESCRIPTION

Embodiments of methods, systems, and apparatus are described through reference to the drawings. Applicant notes that the described embodiments and examples are illustrative and non-limiting. Practical implementation of the features may incorporate a combination of some or all of the aspects, and features described herein should not be taken as indications of future or existing product plans.

In some embodiments, in order to identify and isolate infertile eggs and separate female and male eggs, in-ovo fertility and gender determination processes have been developed using Hyperspectral Imaging (i.e., time-resolved hyperspectral imaging, spatially-resolved hyperspectral imaging), Super-Resolution hyperspectral image processing and analysis, Machine Learning and Deep Learning technologies. The processes were developed based on statistical methods and have shown very promising results on egg fertility and gender determination prior to incubation. Such in-ovo fertility and gender detection systems may include the acquisition of good quality hyperspectral images where the light source may play a role.

In some embodiments, in-ovo fertility and gender determination techniques have been developed based on light scattering patterns through the pre-incubated eggs. The scattered light patterns were then analyzed using gray-level co-occurrence matrix, statistical methods, and Fourier analysis in order to determine the egg fertility and gender of the eggs.

In some embodiments, 'distinguishing features' in the obtained hyperspectral images and/or light scattering patterns have been identified that can consistently differentiate non-fertile eggs from fertile eggs, and male eggs from female eggs.

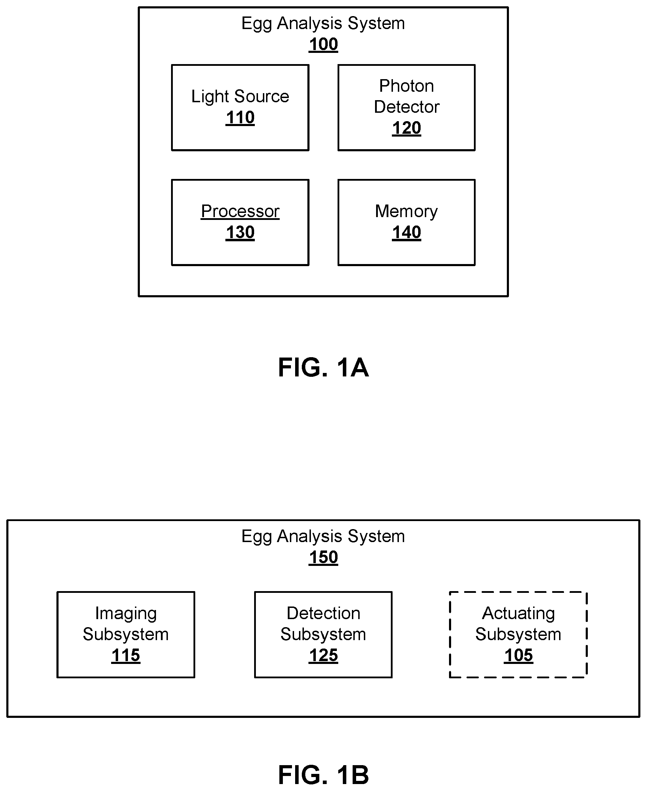

**FIG. 1A** illustrates, in a component diagram, an example of a system for analyzing eggs **100**, in accordance with some embodiments. The system **100** comprises a light source (e.g., a laser) **110**, and at least one photon detector **120**. In some embodiments, eggs may be illuminated by a laser **110** beam (or other light source beam), and the fluctuations of the scattered light detected at a known scattering angle θ by a fast photon detector **120** (e.g., streak camera). Cells in the eggs scatter the light from the laser **110** beam, and the imprint information is detected by the photon detector **120** and used in an analysis that yields information about the cellular particles. The intensity fluctuations of the incident beam is characterized by computing the intensity correlation function, whose analysis provides the diffusion coefficient of the particles (also known as diffusion constant). Since particles of different sizes scatter with different intensities, different scattering angles may be examined in order to determine the optimum angle of detection for the eggs. Other components may be added to the system **100**, including a processor **130** and a memory **140** storing instructions to digitize and analyze the imprint information. For example, the memory **140** may include a digitization module and an analysis module.

**FIG. 1B** illustrates, in a component diagram, another example of a system for analyzing eggs **150**, in accordance with some embodiments. The system **150** includes an imaging subsystem **115**, a detection subsystem **125**, and optionally an actuating subsystem **105**. In some embodiments, the actuating subsystem **105** may be external to the system **150**. The subsystems **115**, **125** and **105** may have separate processors **130** and memory **140**, or alternatively may share a common system **150** processor **130** and memory **140**.

In some embodiments, a light source for an in-ovo fertility and gender detection system **100**, **150** may provide uniform illumination with high intensity to the eggs within the field of view (FOV) of the hyperspectral imaging camera. It should be noted that intensity (also called 'Radiance') is radiant flux emitted, reflected, transmitted or received by a surface, per unit solid angle per unit projected area, while spectral intensity (also called 'spectral radiance') is radiance of a surface per unit wavelength. Intensity is the integral of spectral intensity over the wavelengths of the light source. The following lists more specific specifications for a light source:
1. A light that can cover the field of view (FOV) of the hyperspectral camera (e.g., approximately 16 centimeters (cm) x 2.5 cm, or other dimensions as desired)
2. High spectral intensity at the interested wavelengths range (e.g., at least 120 mW·sr⁻¹·cm⁻²·nm⁻¹, or other spectral radiance as desired)
3. Uniform illumination for each egg within the FOV (e.g., variance < 10%, or as desired)
4. A 'cool' light that produce little heat for a long time run

In addition, the light source may also meet the following specifications in order to be appropriate for industrial use:
5. High stability for a long time run (e.g., 8 hrs or above, or as desired)
6. Long lifetime of the lamp (e.g., >1,000 hrs, or as desired)

In some embodiments, the light source may be part of an imaging subsystem **115**, **200, 250** of a detection system **100**, **150** that includes a line scan spectrograph interconnected with an InGaAs camera (i.e., a hyperspectral imaging camera in the short-wave infrared range), a digital controlled conveyor, and a custom-designed egg holder. The imaging subsystem **115**, **200, 250** works in a transmission mode. Eggs are placed in the egg holder and on the conveyor. The light source in the imaging subsystem is positioned to align with the hyperspectral imaging camera and to provide back illumination for an egg to facilitate the acquisition of hypercube. The light source can be developed based on different lights as long as they are able to meet the above-said specifications.

In some embodiments, the light source is part of an imaging subsystem **115**, **200** that includes a line-scan spectrograph interconnected with an InGaAs camera (i.e., configured to capture images (e.g., spectral images, scattered light). In one example implementation, the spectrograph with a near-infrared spectral range spanning approximately 900 nm to 1700 nm and a spectral resolution of 2.8 nm. In an embodiment, image data is collected in transmission mode. In an embodiment, image data is collected and processed at 100 frames per second. In an embodiment, the imaging system may include a wide field, area scan, snapshot camera.

In some embodiments, one or more light sources **110** in the imaging subsystem **115** may be used to provide back illumination for an egg to facilitate image capture of the fluctuations of scattered light. In one example implementation, a single 250-watt quartz tungsten halogen lamp is used as a light source. Such light source may be connected to an optical fiber (e.g., liquid light guide) to control incident beam size. The controllable beam size and incident beam position using an optical fiber will enhance the precision of algorithm to determine the presence and structure of germinal disc.

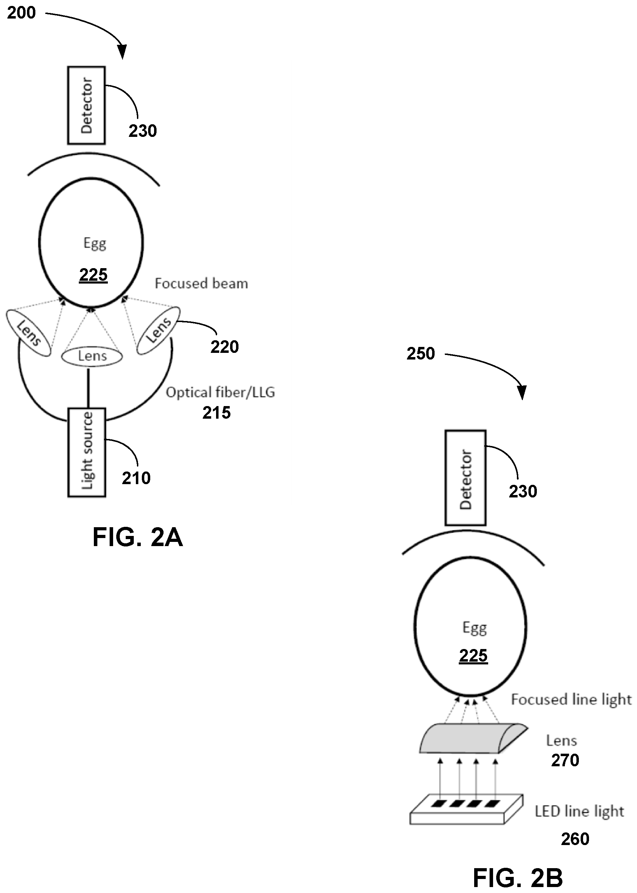

**FIG. 2A** illustrates, in a schematic drawing, an example of a system with optical fiber with incident beam angle for acquiring scattering images of eggs **200**, in accordance with some embodiments. The system comprises a light source **210** connected to one or more lenses **220** via optical fiber/LLG **215**, and a detector **230** receiving the focused beam that has been passed through an egg **225**.

In some embodiments, the light sources **110** may consist of an optical fiber (or a liquid light guide, LLG) and/or a focusing lens to control the size of light beam and incident beam position to generate good scattering images. The size of light beam is critical for the illumination system. A larger beam may give higher light throughput and a larger scattering area, but it can be difficult to quantify the light scattering characteristics. A smaller beam, albeit desirable for scattering measurement, but it may have lower efficiency for the camera that can result in lower SNR (i.e., signal-to-noise ratio) images. The selected beam size should be a trade-off between the light efficiency and scattering measurement. In one example implementation, a liquid light guide (LLG) with a core diameter of 7.6 mm and 0.52 numerical aperture is used to guide the incident light to the illumination position that may be right underneath the egg. A focusing lens may be placed between the end of LLG and the egg in order to form a sharp and focused incident beam at the egg. Multiple optical fibers and focusing lens may be used to provide higher light throughput in order to have a good balance between the light efficiency and scattering measurement. In this case, the incident angles of the fibers/LLGs may be adjusted within a small range (less than 30∘) with equal intervals.

In some embodiments, an optimal light source based on halogen lamps (e.g., quartz tungsten halogen, QTH) may consist of 2 or 3 identical light source units each of which provide back illumination for a column of eggs. A QTH light source unit may include a 250 W QTH lamp, a light source housing, a lamp power supply, a fiber bundle focusing assembly, a liquid light guide, a light guide collimating probe, and all the necessary interconnection cables and parts for operation.

The QTH lamp may be operated in current, power, or intensity control mode to meet the operational requirements. The lamp power supply is highly regulated for maximum stability. The QTH housing offers a built-in ignitor board and rear reflector for optimum lamp performance. A built-in cooling fan maintains a safe operating temperature for the lamp. The fiber bundle focusing assembly that is connected to the light housing is used to convert a collimated light source into a fiber optic source, i.e., a liquid light guide (LLG) that is connected to the focusing assembly. The liquid light guide carries light from the housing to a position underneath the egg holder where it is aligned with the hyperspectral imaging camera. The light guide collimating probe that is connected to the LLG collimates the highly divergent outputs of LLG and provides back illumination for an egg to acquire high quality of hypercube.

**FIG. 2B** illustrates, in a schematic drawing, an example of a system with LED line light for acquiring scattering images of eggs **250**, in accordance with some embodiments. The system comprises an LED line light source **260**, a lense **270**, and the detector **230** receiving the focused beam that has been passed through the egg **225**.

Light-emitting diodes (LEDs) are semiconductors that convert electrical energy into light energy. Compared to QTH lamps, LED lamps can provide much higher light output intensity, need lower energy consumption, have much longer shelf life without decreasing light intensity, and produce much less heat. However, unlike a QTH lamp that produces a continuous spectrum of light from near ultraviolet to the infrared, there are only a few wavelengths that are available for LED lamps especially in the IR/SWIR range.

Several wavelengths that were identified to be useful for the egg fertility and gender determination include approximately 860 nm, 1050 nm, and 1250 nm. An LED line light was designed to accommodate the LED units with identified wavelengths at a carefully tuned ratio that can produce equal light output intensity level at all wavelengths. The spectral intensity of the light output, i.e., the radiance of a surface per unit wavelength should be at least: $120 mW ⋅ {sr}^{- 1} ⋅ {cm}^{- 2} ⋅ {nm}^{- 1} .$ Depending on the light output intensity level of LED units, the LED light source may work in strobe mode to over-drive LEDs to obtain a boost in their performance. If the LED light source works in strobe mode, the LEDs at all wavelengths should do strobing simultaneously. The LED line light should be mounted underneath the egg holder and aligned with the FOV of the HSI camera to provide back illumination for eggs to acquire good quality of hypercubes.

In some embodiments, the photon detector **120**, **230** is part of a detection subsystem **125** that analyses received scattering data to detect cellular particles in an egg, including, for example, a germinal disc.

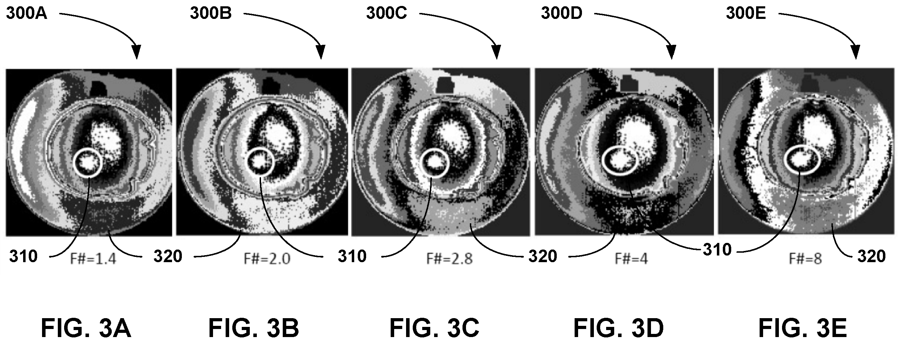

**FIGs. 3A to 3E** illustrate scan images **300A** to **300E** of a germinal disc **310** in an egg **320**, in accordance with some embodiments. Detection of the germinal disc **310** was performed in the light scattering images **300A** to **300E** that were obtained with different aperture sizes (F#) of the camera. The pattern of germinal disc **310** appeared as a round disc in light scattering images **300A** to **300E**. The egg handling protocol ensures that the germinal disc **310** moves to the top of the egg **320** in the field of view of the camera during scanning. Although the germinal disc **310** was able to be identified in all light scattering images, image **300C** with F# of 2.8 outperformed other scattering images with the best contrast and separability.

In some embodiments, system **100** may optionally include an actuating system **105** to actuate a conveyor configured to move an egg into the field of view of the system's photon detector **120**, **230** (e.g., camera) optics. In one example implementation, the conveyor is a Dorner 2200 series conveyer system comprises a belt, a roller, a stepping motor, and a programmable logic controller (PLC) touch screen. The speed of the conveyor may be adjustable. For example, the speed of the conveyor may be adjusted based on the speed of the photon detector **120**, **230** (e.g., the frame rate of camera optics) to minimize image distortion (e.g., motion blur). The speed of the conveyor may also be adjusted based on other factors, e.g., desired detection throughput, the working distance between detector and object, the desired illumination level at the object.

The conveyor may include trays or racks adapted to receive eggs therein. In some embodiments, the trays or rack may then be stored (either on or off the conveyor) while maintaining each egg in a given position (e.g., a vertical position, a first angled position, a second angled position, etc.). In some embodiments, the rack may rotate such that the eggs therein are maintained in a different positon (e.g., a vertical position, a first angled position, a second angled position, etc.).

In an embodiment, the conveyor may be configured to present multiple eggs (e.g., two eggs, four eggs, etc.) to be imaged simultaneously. Accordingly, in this embodiment, each spectral image or scattering data may include data for multiple eggs, and each such image or scattering data may be segmented during processing to isolate scattering data for each egg. Processor **130** may be configured to send control commands to conveyor to control its movement.

The imaging subsystem **115**, **200**, **250** may be interconnected with a detection subsystem **125** by way of a conventional serial or parallel interface. In an embodiment, imaging subsystem **115**, **200**, **250** may be interconnected with detection subsystem **125** by way of a network comprising wired links, wireless links, or a combination thereof. In this embodiment, one or both of imaging subsystem **115**, **200**, **250** and detection subsystem **125** may include a suitable network interface and/or network transceivers. In some embodiments, the detection subsystem **125** may be a cloud service which receives scattering data for an egg as input, and provides a sex and/or fertility of the egg as output.

In some embodiments, the detection subsystem **125** or system **100** may connect to an actuating subsystem **105** to trigger actuation of apparatuses based on results computed by the detection subsystem **125**. The actuating subsystem **105** is operable to transmit a control signal to actuate an apparatus according to the classified unhatched egg. The actuating subsystem **105** is operable to generate data signals for the gender and fertility of the unhatched egg, for example. The actuating subsystem **100** is operable to transmit the output data signals to hardware or apparatus to trigger actuation thereof. For example, the actuating subsystem **105** may move or separate the unhatched egg.

In some embodiments, an actuating subsystem **105** may receive data signals of classification results from the detection subsystem **125** and removes the undesired eggs (non-fertile and/or male) from the assembly line using one or more apparatuses that are in physical contact with the eggs or otherwise can trigger movement or separation of eggs. For example, actuating subsystem **105** may include or interface with one or more robotic arms with end effectors (robotic hands) that may be used to grasp and drop or replace eggs which are indicated by the classification signals from detection subsystem as non-fertile and/or male eggs. There may be other apparatuses that can separate or move eggs based on the classification signals from detection subsystem and this is an illustrative example only. Accordingly, the actuating subsystem **105** triggers actuation of hardware components based on the classification signals from detection subsystem **125**. In example embodiments the actuation may involve physical movement of the eggs to separate the eggs into different streams, for example. As another example a conveyer may be triggered or controlled to move eggs. Detection subsystem **125** generates output signals for actuating subsystem **105** to provide control commands to trigger actuation of various apparatuses.

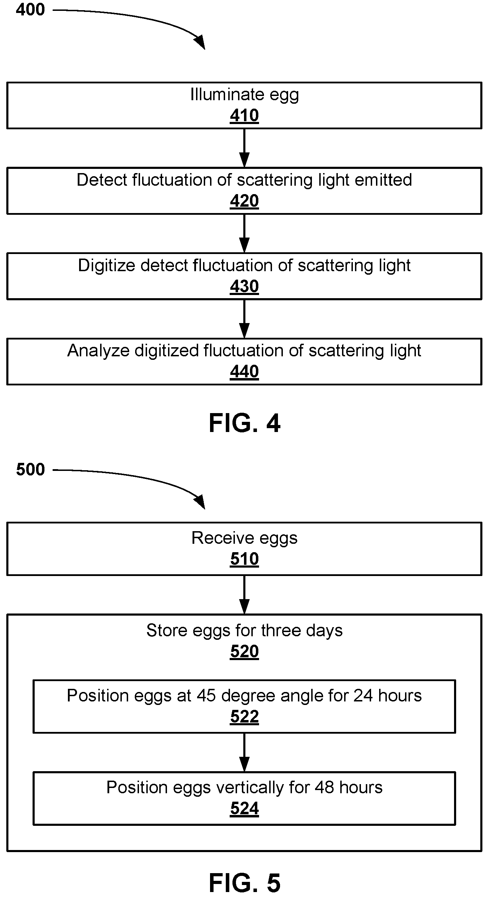

**FIG. 4** illustrates, in a flowchart, an example of a method of analyzing eggs **400**, in accordance with some embodiments. The method **400** comprises illuminating **410** an egg using a light source (e.g., a laser) **110** beam, and detecting **420** fluctuations of scattered light emitting from the egg using a fast photon detector **120**. Next the fluctuations, or imprint information, detected by the photon detector **120** is digitized **430**, and the digitized information analysed **440** for cellular particulars. For example, an intensity correlation function may be performed to determine the diffusion coefficients of the particles (i.e., diffusion constant). Other steps may be performed by the method **400**, including determining an optimal angle of detection for the egg.

The shape and size of the germinal disc which floats on the egg yolk is different between fertile and non-fertile eggs. The germinal disc in a fertile egg (also called blastoderm) is visually seen as a symmetrical circular ring, while the germinal disc in a non-fertile egg (also called blastodisc) looks like an asymmetrical solid spot. There have been several studies to identify possible gender differentiating features prior to incubation. It is known that there could be about 40,000 to 60,000 blastoderm cells available in the germinal disc of chicken egg. Female birds are heterogametic with one Z and one W sex chromosome, whereas male birds have two Z chromosomes. The Z chromosome has approximately threefold higher DNA content than the W chromosome. Therefore, the total DNA amount in cells from male birds will be higher than in cells from female birds.

Application of multivariate methods for spectral feature selection has produced promising results in such fields as tumor identification or classification of different cell types. Further, considering that the germinal disc of the male egg is denser than that of the female egg, the spectral scattering patterns are expected to be different and could explain the statistical difference observed by the spectral and image features extracted from the hyperspectral images. Therefore, the distinguishing features for fertility and gender detection may be discovered based on light scattering measurement.

Light scattering happens when light "hits" a small object (a particle or a molecule) and thereby changes its direction. The ability of scattering particles to scatter light depends on their densities and sizes. It is a process when incident light of energy is absorbed by an object and subsequently light of energy is emitted. The angle, frequency and intensity (i.e., power) of light scattering can be measured to determine the particle size and density of materials. Angle-resolved scattering measurements capture light as a function of the scattering angle, and invert the angles to deduce the average size of the scattering objects via a computational light scattering model such as Mie scattering theory, which predicts angles based on the size of the scattering sphere. Combining these techniques allows for a measurement of average scatter size and average particle size of the object. In addition, multiple scattering properties that can be calculated by averages of single sphere scattering efficiencies obtained from Mie scattering theory are also used to predict the structure of the object. Therefore, egg fertility and gender detection methods have been developed based on the predicted size and structure of germinal disc using different light scattering measurements and patterns.

The light scattering may vary with particle sizes and/or densities. The intensity of the scattered light in different directions may strongly depend on the wavelength of incident light and the size, shape and composition of particles. The effectiveness of scattering depends on the size parameter *x* that is defined as $x = \frac{2 π . r}{λ}$, where r is the radius of a spherical particle (or particle size) and λ is the wavelength of incident light. A germinal disc normally having 40,000-60,000 blastoderm cells appears as either a single small white mass for unfertile eggs or a double ring and larger white disk for fertile eggs. The diameter of a germinal disc may vary from less than 1mm up to 4 mm. This indicates that the size parameter of a germinal disc is greater than 1,500, which means that the Mie scattering (for x=0.2 to 2000) or geometric optics (for x>2000) may be valid depending on the size of the GD. The light intensity in scattering image can be a function of the scattering angles and the angles can be inverted to deduce the average size of the scattering objects, i.e., the scattering size. The scattering size may also be measured with the scattering cross-section that can be determined by dividing the power of the scattered light by the intensity of the incident light.

As noted above, in some embodiments, prediction of egg fertility and gender of non-incubated eggs is based on identifying and characterizing light scattering patterns of the egg's germinal disc (or other cells in mitochondrial DNA). Therefore, the germinal disc should be in the camera's field of view (FOV) during scanning. Eggs may be scanned with its "big" (i.e., large) end upwards. The "big" or "large" end is considered to be the larger in diameter of the two ends of an egg. What is desired is to ensure that the germinal disc is located on top of the big/large end of the egg in order to appropriately record and measure its light scattering patterns. Normally the germinal disc in the egg could be located randomly anywhere on the surface of the egg's yolk. In some embodiments, a protocol of egg handling provides that the germinal disc is located on top of the egg (big/large end) and in the camera's field of view.

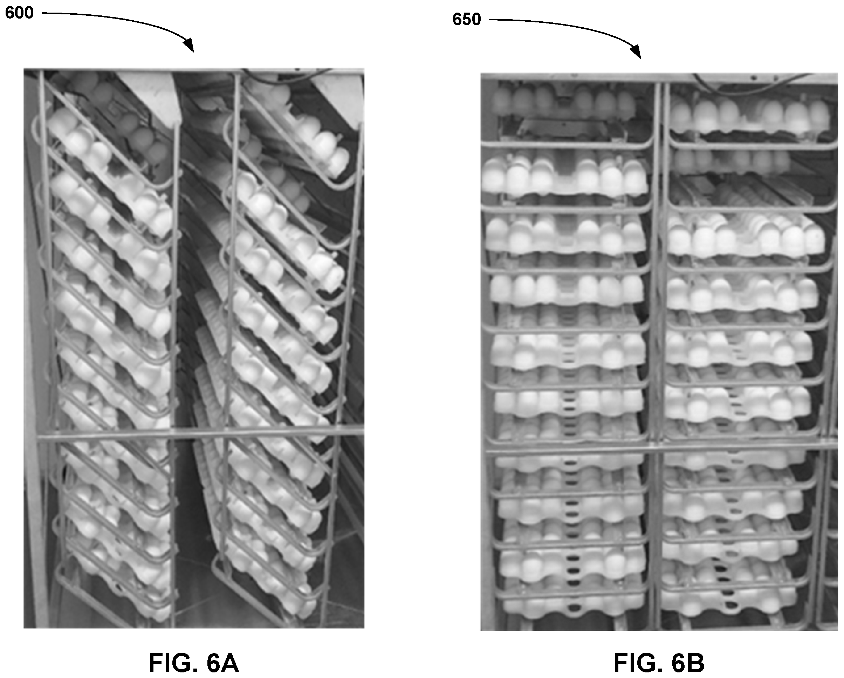

**FIG. 5** illustrates, in a flowchart, an example of a protocol (e.g., method) of handling an egg such that its germinal disc is located on the top (large end) of the egg **500**, in accordance with some embodiments. The method **500** may be performed by an egg processing system. Eggs may be placed in a tray **510** with their big end upwards. For example, an egg processing system may be configured to receive eggs in a manner that causes the eggs to fall into place in a rack of an egg holding apparatus. Next, the eggs may be stored **520** at approximately between 18 to 20 degrees Celsius for at least three days. The eggs (e.g., the rack holding the eggs) may be positioned **522** at a first angle (e.g., approximately a 45 degree angle) for a first period (e.g., the first approximately 24 hours). For example, an actuator of an egg processing system may rotate the rack at approximately a 45 degree angle. **FIG. 6A** illustrates an example of eggs positioned at 45 degree angle **600**, in accordance with some embodiments. Next the eggs (e.g., the rack holding the eggs) are positioned **524** at a second angle (e.g., flat; the rack may be in a horizontal positon such that the eggs are in a vertical position with the "big" end up") for a second period (e.g., the next approximately 48 hours). For example, a timer may indicate that the rack has been at approximately 45 degrees for a first time period (e.g., approximately 24 hours) which triggers the actuator to rotate the rack to a horizontal (flat) position. Alternatively, the racks may be set to rotate at pre-determined times. It should be understood that the eggs may be left in the approximately 45 degree angle for longer than 24 hours and then in the vertical position (i.e., flat rack) for longer than 48 hours. Alternatively, the rack or tray may hold the eggs at a desired first angle when the rack or tray is flat, and then be rotated to an angel such that the eggs are then in a vertical position in the rack or tray. Other, angles for racks and trays may be used. **FIG. 6B** illustrates an example of eggs positioned in a vertical position **650**, in accordance with some embodiments. At this point, the germinal disc should be located at the top (large end) of the egg.

An experiment was conducted to investigate the effectiveness of this protocol **500** on the germinal disc location in an egg. A total of 100 freshly laid fertile eggs were received from a local egg farm over the course of a month. Upon arrival the eggs were stored for three days. Fifty eggs were stored according to the protocol, while the remaining 50 eggs were stored only in the vertical direction with the big end up at the same room temperature for three days. After the three-day storage, the eggshell of each egg was carefully peeled from the center of the egg top (large end) to the side of the boundary defined by the camera's FOV to assess the location of the corresponding germinal disc. For the eggs that were stored as per the protocol, 43 out of 44 eggs (98%) showed the germinal disc right on top of the eggs upon opening and examining the eggs. For the eggs that were stored for three days without positioning at 45 degrees, 38 out of 43 eggs (88%) had their germinal disc on top of the eggs. Thus, the experiment showed that the likelihood of locating germinal disc on top of the egg and in the camera's FOV can be increased by applying the egg handling protocol **500**. Therefore, the protocol **500** may improve the performance of egg fertility and gender detection methods that are developed based on detection of light scattering characteristics of the germinal disc.

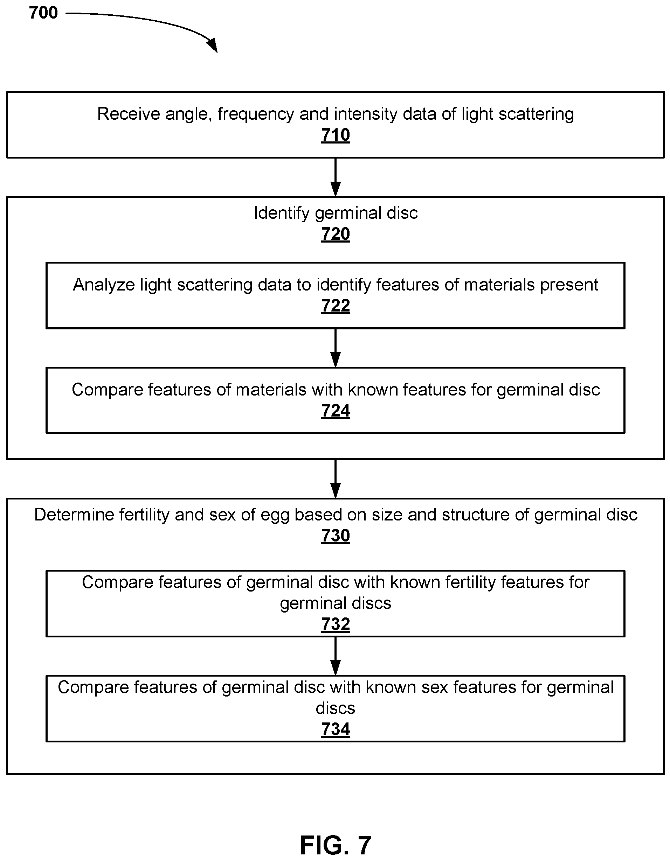

**FIG. 7** illustrates, in a flowchart, an example of a method of analyzing light scattering **700**, in accordance with some embodiments. The method comprises receiving **710** angle, frequency and intensity data of light scattering obtained from a photon detector **120** from an egg illuminated using a light source **110**. For example, eggs may be placed in an egg holder and on a conveyor. The light source **110** may be positioned to align with the photon detector **120** (e.g., HSI camera). The light source **110** will illuminate the egg at the small end, while the big/large end of the egg will be in the FOV of the photon detector. The light scattering data may then be analyzed **720** to identify the germinal disc. The light scattering data is analyzed **722** to identify features of materials present. Next, features (e.g., size, shape, orientation, etc.) of each material in the light scattering data is compared **724** with known features of germinal discs to identify the germinal disc in the light scattering data from any other material or particle present. Next, the fertility and/or sex of the egg is determined **730** based on the size and structure of the identified germinal disc. Features of the identified germinal disc are compared **732** with known fertility features for germinal discs. For example, in a non-fertile egg, the scattering image data of the germinal disc egg may show features that are related to blastodisc which looks like a white pimple; while in a fertile egg, the scattering image data of the germinal disc may show features that are related to blastoderm which looks like a white hollow disc with a white pimple at the centre. Similarly, features of the identified germinal disc are compared **734** with known sex features for germinal discs. Thus, the identified germinal disc data is compared to known germinal disc features to distinguish between fertile and non-fertile eggs, and between male and female eggs. Other steps may be added to the method, including following germinal disc placement protocol **500**, and separating distinguished eggs based on egg type using a conveyor subsystem.

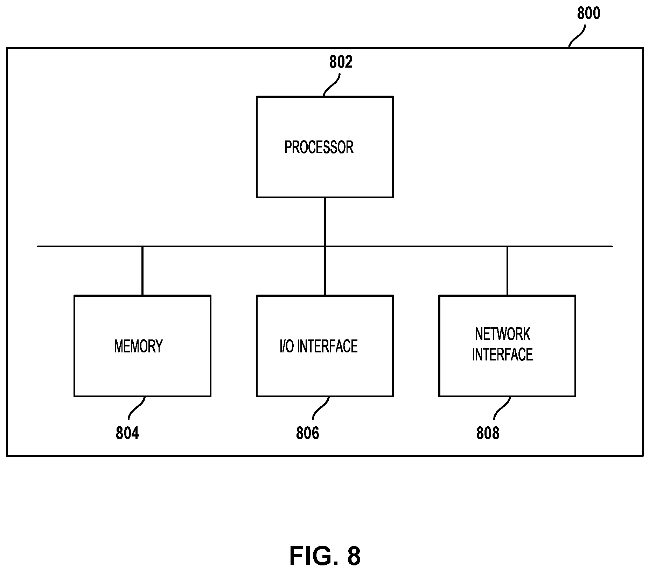

**FIG. 8** is a schematic diagram of a computing device **800** such as a server or other computer in a device. As depicted, the computing device includes at least one processor **802**, memory **804**, at least one I/O interface **806**, and at least one network interface **808**.

Processor **802** may be an Intel or AMD x86 or x64, PowerPC, ARM processor, or the like. Memory **804** may include a suitable combination of computer memory that is located either internally or externally such as, for example, random-access memory (RAM), read-only memory (ROM), compact disc read-only memory (CDROM).

Each I/O interface **806** enables computing device **800** to interconnect with one or more input devices, such as a keyboard, mouse, camera, touch screen and a microphone, or with one or more output devices such as a display screen and a speaker.

Each network interface **808** enables computing device **800** to communicate with other components, to exchange data with other components, to access and connect to network resources, to serve applications, and perform other computing applications by connecting to a network (or multiple networks) capable of carrying data including the Internet, Ethernet, plain old telephone service (POTS) line, public switch telephone network (PSTN), integrated services digital network (ISDN), digital subscriber line (DSL), coaxial cable, fiber optics, satellite, mobile, wireless (e.g. Wi-Fi, WiMAX), SS7 signaling network, fixed line, local area network, wide area network, and others.

The foregoing discussion provides example embodiments of the inventive subject matter. Although each embodiment represents a single combination of inventive elements, the inventive subject matter is considered to include all possible combinations of the disclosed elements. Thus, if one embodiment comprises elements A, B, and C, and a second embodiment comprises elements Band D, then the inventive subject matter is also considered to include other remaining combinations of A, B, C, or D, even if not explicitly disclosed.

The embodiments of the devices, systems and methods described herein may be implemented in a combination of both hardware and software. These embodiments may be implemented on programmable computers, each computer including at least one processor, a data storage system (including volatile memory or non-volatile memory or other data storage elements or a combination thereof), and at least one communication interface.

Program code is applied to input data to perform the functions described herein and to generate output information. The output information is applied to one or more output devices. In some embodiments, the communication interface may be a network communication interface. In embodiments in which elements may be combined, the communication interface may be a software communication interface, such as those for inter-process communication. In still other embodiments, there may be a combination of communication interfaces implemented as hardware, software, and combination thereof.

Throughout the foregoing discussion, numerous references will be made regarding servers, services, interfaces, portals, platforms, or other systems formed from computing devices. It should be appreciated that the use of such terms is deemed to represent one or more computing devices having at least one processor configured to execute software instructions stored on a computer readable tangible, non-transitory medium. For example, a server can include one or more computers operating as a web server, database server, or other type of computer server in a manner to fulfill described roles, responsibilities, or functions.

The technical solution of embodiments may be in the form of a software product. The software product may be stored in a non-volatile or non-transitory storage medium, which can be a compact disk read-only memory (CD-ROM), a USB flash disk, or a removable hard disk. The software product includes a number of instructions that enable a computer device (personal computer, server, or network device) to execute the methods provided by the embodiments.

The embodiments described herein are implemented by physical computer hardware, including computing devices, servers, receivers, transmitters, processors, memory, displays, and networks. The embodiments described herein provide useful physical machines and particularly configured computer hardware arrangements.

Although the embodiments have been described in detail, it should be understood that various changes, substitutions and alterations can be made herein.

Moreover, the scope of the present application is not intended to be limited to the particular embodiments of the process, machine, manufacture, composition of matter, means, methods and steps described in the specification.

As can be understood, the examples described above and illustrated are intended to be exemplary only.

## Claims

1. A method comprising:
obtaining, from at least one photon detector, light scattering data comprising angle, frequency and intensity data of scattered light emitted from an egg illuminated by at least one light source;
analyzing the light scattering data to identify a germinal disc of the egg; and
determining a fertility of the egg, a sex of the egg or both the fertility and the sex of the egg based on at least one of a size and a structure of the identified germinal disc.

2. The method of claim 1, wherein analyzing the light scattering data to identify the germinal disc of the egg comprises distinguishing the germinal disc of the egg from other materials or particles of the egg represented by the light scattering data.

3. The method of claim 1 or 2, wherein analyzing the light scattering data to identify the germinal disc of the egg comprises:
analyzing the light scattering data to identify at least one feature of material present; and
comparing the identified at least one feature with at least one known germinal disc feature.

4. The method of claim 3, wherein the at least one feature of material present comprises at least one of a size, a shape and an orientation of at least one material of the egg represented by the light scattering data.

5. The method of any one of claims 1 to 4, wherein determining the fertility of the egg, the sex of the egg or both the fertility and the sex of the egg based on at least one of the size and the structure of the identified germinal disc comprises comparing at least one feature of the identified germinal disc with at least one known feature of germinal discs.

6. The method of claim 5, wherein the at least one known feature of germinal discs comprises at least one known fertility feature of germinal discs, at least one known sex feature of germinal discs or both at least one known fertility feature of germinal discs and at least one known sex feature of germinal discs.

7. The method of any one of claims 1 to 6, further comprising prior to obtaining the light scattering data, handling the egg to position the germinal disc within a field of view of the at least one photon detector.

8. The method of claim 7, wherein the handling comprises:
rotating the egg to position the egg at a first angle;
holding the egg positioned at the first angle for a first period of time;
after holding the egg positioned at the first angle for the first period of time, rotating the egg to position the egg at a second angle; and
holding the egg positioned at the second angle for a second period of time.

9. The method of claim 8, wherein the first angle is approximately 45°.

10. The method of claim 8 or 9, further comprising storing the egg for a predefined amount of time prior to rotating the egg to position the egg at the first angle.

11. The method of any of one of claims 1 to 10, wherein the method is carried out on a plurality of eggs, and further comprising separating eggs that are determined to be infertile from eggs that are determined to be fertile, and wherein optionally the method further comprises separating eggs that are determined to be female from eggs that are determined to be male.

12. The method of any of one of claims 1 to 10, wherein the method is carried out on a plurality of eggs, and further comprising separating eggs that are determined to be female from eggs that are determined to be male.

13. The method of any one of claims 1 to 12, further comprising digitizing the light scattering data prior to analyzing the light scattering data.

14. The method of any one of claims 1 to 12, wherein the light scattering data obtained from the at least one photon detector is digitized light scattering data.

15. The method of any one of claims 1 to 14, wherein the at least one photon detector comprises a hyperspectral imaging camera.
